# EUROPEAN PATENT APPLICATION

(11) **EP 4 830 631 A2**
(43) Date of publication of application: **28.10.2026**
(21) Application number: 26203911.8
(22) Date of filing: 19.08.2021
(51) Int. Cl.: A61B 5/327

(54) **ELECTROCARDIOGRAM LEAD RECONSTRUCTION USING MACHINE LEARNING**

(30) Priority: 28.08.2020 US 202063071803 P
(62) Document number(s) of the earlier application(s) in accordance with Art. 76 EPC: 21769657.4 / 4 203 792
(71) Applicant: Analog Devices International Unlimited Company, Co. Limerick (IE)
(72) Inventor: GRANDE, Alejandro, Wilmington, 01887 (US); CALPE MARAVILLA, Javier, Wilmington, 01887 (US); REDON SEGRERA, Monica, Wilmington, 01887 (US); GOPINATHAN, Venugopal, Wilmington, 01887 (US); AKL, Tony, Wilmington, 01887 (US)
(74) Representative: Strange, Harry George

(57) **Abstract**

A method for reconstructing 12-lead standard electrocardiogram (ECG) system signals using an *M* lead system, the method comprising recording signals acquired by the 12-lead standard ECG system; recording signals acquired by the *M*-lead system; and using the recorded signals to train a machine learning model to produce the reconstructed 12-lead standard ECG system signals using the *M*-lead system.

## Description

### CROSS-REFERENCE TO RELATED APPLICATION

This patent application claims the benefit of the filing date of U.S. Provisional Patent Application Serial No. 63/071,803, filed on August 28, 2020, and entitled "ELECTROCARDIOGRAM LEAD RECONSTRUCTION USING MACHINE LEARNING," the content of which is hereby expressly incorporated by reference in its entirety.

### FIELD OF THE DISCLOSURE

This disclosure relates generally to the field of electrocardiography and, more particularly, to systems and methods for electrocardiogram (ECG) lead reconstruction using machine learning.

### BACKGROUND

An ECG is a graph of voltage over time of electrical activity of the heart of a subject using electrodes placed on the subject's skin. The electrodes detect small electrical changes resulting from cardiac muscle depolarization followed by repolarization during each cardiac cycle, or heartbeat. Irregular ECG patterns may indicate a variety of cardiac abnormalities. In a conventional 12-lead ECG system, 10 electrodes are placed in standard locations on the subject's torso and limbs. The overall magnitude of the electrical potential of the subject's heart is then measured from twelve different angles, or "leads," and is recorded over a period of time (e.g., 10 seconds) to capture the overall magnitude and direction of the heart's electrical activity throughout the cardiac cycle.

The three primary components of an ECG include the P wave, which corresponds to the depolarization of the atria, the QRS complex, which corresponds to the depolarization of the ventricles coupled to the repolarization of the atria which is relatively small in amplitude, and the T wave, which represents the repolarization of the ventricles. During each heartbeat, a healthy heart has an orderly progression of depolarization, which gives rise to a characteristic ECG tracing. An ECG may convey a great deal of information about the structure and electrical function of the heart and is therefore a useful diagnostic tool.

### BRIEF DESCRIPTION OF THE DRAWINGS

To provide a more complete understanding of the present disclosure and features and advantages thereof, reference is made to the following description, taken in conjunction with the accompanying FIGURES, wherein like reference numerals represent like parts, in which:
FIGs. 1A and 1B are block diagrams illustrating various features of a system for ECG lead reconstruction in accordance with features of embodiments described herein;
FIG. 2A illustrates an artificial neural network (ANN) for use in a system for ECG lead reconstruction in accordance with features of embodiments described herein;
FIG. 2B illustrates an alternative embodiment of an ANN for use in a system for ECG lead reconstruction in accordance with features of embodiments described herein;
FIGs. 3A-3B collectively illustrate a manner in which an output of a regressor in response to previously unknown or untrained events may be improved using expert committees in accordance with features of embodiments described herein for implementing a system for ECG lead reconstruction;
FIG. 4 illustrates a manner in which a quality of a reconstruction developed using a system for ECG lead reconstruction may be evaluated, or assessed, on-the-fly based on intrinsic characteristics of the relationships among selected signals of a 12-lead standard ECG system in accordance with features of embodiments described herein;
FIG. 5 illustrates a manner in which weights of a regressor may be adapted to improve a quality of reconstruction provided by a system for ECG lead reconstruction as evaluated, or assessed, based on intrinsic characteristics of relationships among various leads of a 12-lead standard ECG system in accordance with features of embodiments described herein;
FIG. 6 illustrates a manner in which a ranked list of various of lead configurations specific to a particular human subject may be generated based on the mathematical accuracy of the lead configurations in accordance with features of embodiments described herein;
FIG. 7 illustrates an example ranking technique that may be used for ranking various lead configurations specific to a particular human subject in accordance with features of embodiments described herein;
FIGs. 8A-8C collectively illustrate an alternative technique for ECG lead reconstruction based on *a priori* clustering of input data using fuzzy c-means (FCM) in accordance with features of an alternative embodiment described herein;
FIGs. 9A and 9B are flow diagrams illustrating a technique for ECG lead reconstruction in accordance with features of embodiments described herein;
FIG. 10 is a flow diagram illustrating a technique for on-the-fly analysis of an ECG lead reconstruction system in accordance with features of embodiments described herein;
FIG. 11 is a block diagram of a computer system that may be used to implement all or some portion of the system for ECG lead reconstruction in accordance with features of embodiments described herein

### DESCRIPTION OF EXAMPLE EMBODIMENTS

For the purposes of the present disclosure, the phrase "A and/or B" means (A), (B), or (A and B). For the purposes of the present disclosure, the phrase "A, B, and/or C" means (A), (B), (C), (A and B), (A and C), (B and C), or (A, B, and C). The term "between," when used with reference to measurement ranges, is inclusive of the ends of the measurement ranges. When used herein, the notation "A/B/C" means (A), (B), and/or (C).

The description uses the phrases "in an embodiment" or "in embodiments," which may each refer to one or more of the same or different embodiments. Furthermore, the terms "comprising," "including," "having," and the like, as used with respect to embodiments of the present disclosure, are synonymous. The disclosure may use perspective-based descriptions such as "above," "below," "top," "bottom," and "side"; such descriptions are used to facilitate the discussion and are not intended to restrict the application of disclosed embodiments. The accompanying drawings are not necessarily drawn to scale. Unless otherwise specified, the use of the ordinal adjectives "first," "second," and "third," etc., to describe a common object, merely indicate that different instances of like objects are being referred to and are not intended to imply that the objects so described must be in a given sequence, either temporally, spatially, in ranking or in any other manner.

In the following detailed description, reference is made to the accompanying drawings that form a part hereof, and in which is shown, by way of illustration, embodiments that may be practiced. It is to be understood that other embodiments may be utilized, and structural or logical changes may be made without departing from the scope of the present disclosure. Therefore, the following detailed description is not to be taken in a limiting sense.

The following disclosure describes various illustrative embodiments and examples for implementing the features and functionality of the present disclosure. While particular components, arrangements, and/or features are described below in connection with various example embodiments, these are merely examples used to simplify the present disclosure and are not intended to be limiting. It will of course be appreciated that in the development of any actual embodiment, numerous implementation-specific decisions must be made to achieve the developer's specific goals, including compliance with system, business, and/or legal constraints, which may vary from one implementation to another. Moreover, it will be appreciated that, while such a development effort might be complex and time-consuming; it would nevertheless be a routine undertaking for those of ordinary skill in the art having the benefit of this disclosure.

In the Specification, reference may be made to the spatial relationships between various components and to the spatial orientation of various aspects of components as depicted in the attached drawings. However, as will be recognized by those skilled in the art after a complete reading of the present disclosure, the devices, components, members, apparatuses, etc. described herein may be positioned in any desired orientation. Thus, the use of terms such as "above", "below", "upper", "lower", "top", "bottom", or other similar terms to describe a spatial relationship between various components or to describe the spatial orientation of aspects of such components, should be understood to describe a relative relationship between the components or a spatial orientation of aspects of such components, respectively, as the components described herein may be oriented in any desired direction. When used to describe a range of dimensions or other characteristics (e.g., time, pressure, temperature, length, width, etc.) of an element, operations, and/or conditions, the phrase "between X and Y" represents a range that includes X and Y.

Further, the present disclosure may repeat reference numerals and/or letters in the various examples. This repetition is for the purpose of simplicity and clarity and does not in itself dictate a relationship between the various embodiments and/or configurations discussed. Example embodiments that may be used to implement the features and functionality of this disclosure will now be described with more particular reference to the accompanying FIGURES.

Embodiments described herein comprise systems and methods of reconstructing a standard 12-lead ECG tracing (using 10 electrodes) by means of two or more leads using machine learning. In accordance with features of embodiments described herein, one aspect of the systems and methods involves optimization of placement of the electrodes on the torso and/or limbs of particular human subject to ensure the highest quality reconstruction for that particular individual. Additionally and/or alternatively, a local qualitative confidence value of the reconstruction may be provided for validating performance of the system.

The 10 electrodes in a 12-lead standard ECG system are set forth in Table 1 below.

| **Electrode Name** | **Electrode Placement** |
|---|---|
| RA | On the right arm, avoiding thick muscle. |
| LA | On the left arm in the same location as RA. |
| RL | On the right leg, lower end of inner aspect of calf muscle, avoiding bony prominences. |
| LL | On the left leg in the same location as RL. |
| V₁ | In the fourth intercostal space just to the right of the sternum. |
| V₂ | In the fourth intercostal space just to the left of the sternum. |
| V₃ | Between leads V₂ and V₄. |
| V₄ | In the fifth intercostal space in the mid-clavicular line. |
| V₅ | Horizontally even with V₄, in the left anterior axillary line. |
| V₆ | Horizontally even with V₄ and V₅ in the mid-axillary line. |

The 12 leads of the 12-lead standard ECG system include limb leads I, II, and III; augmented limb leads aVR, aVL, and aVF; and precordial leads V₁, V₂, V₃, V₄, V₅, and V₆. In the 12-lead standard ECG system, each lead corresponds to one or a combination of the electrodes. For example: $I = LA - RA$ $II = LL - RA$ $III = LL - LA$

For purposes of example only, the ECG reconstruction system and methods described herein may be explained with reference to an *M-*lead system (formed using *X* electrodes) in combination with an artificial neural network (ANN), in which *M* is equal to three and *X* is equal to four; however, it will be recognized that more or fewer leads and corresponding electrodes and machine learning techniques other than ANN may be used without departing from the spirit or scope of embodiments described herein.

One example embodiment is a technique for replicating an ECG signal produced by a 12-lead standard ECG system (formed by 10 electrodes) by means of a 3-lead system (formed by four electrodes) in combination with an artificial neural network (ANN) comprising a trained model. The positioning of the electrodes for implementing the 3-lead system may be personalized for a particular human subject during training of the model. As a result, the 3-lead system can be used to implement an ambulatory ECG system without sacrificing the accuracy afforded by the standard 12-lead ECG system.

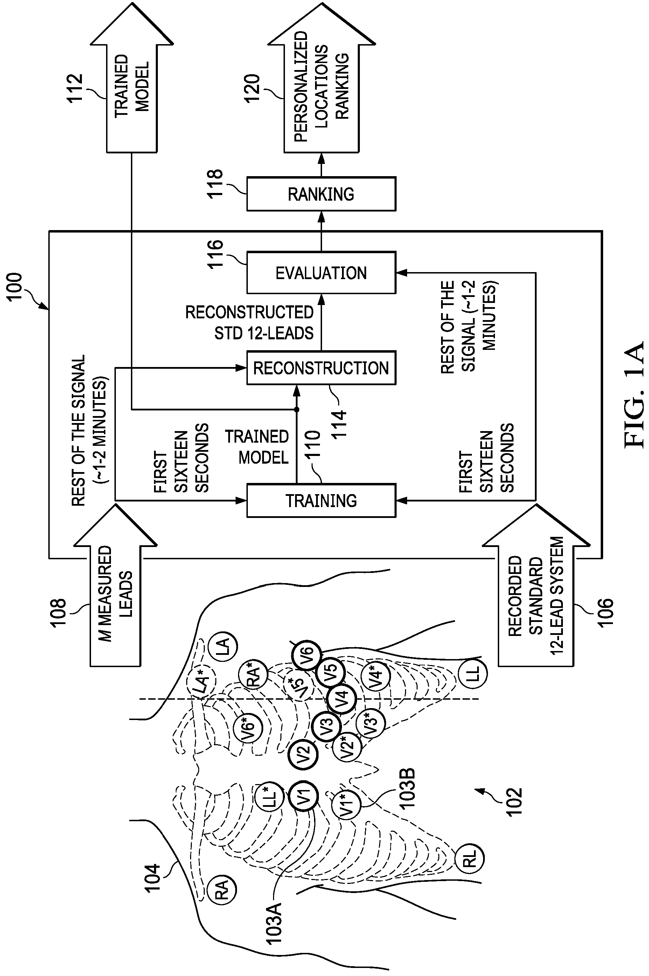

FIG. 1A illustrates a functional block diagram of a system 100 for performing ECG lead reconstruction using a subset of *M* leads of an enhanced ECG system 102. In accordance with embodiments described herein, the enhanced ECG system 102 includes the electrodes of a 12-lead standard ECG system (represented in FIG. 1A by an electrode 103A) as well as additional electrodes (represented in FIG. 1A by an electrode 103B). Although as shown in FIG. 1A, the enhanced ECG system 102 includes nine additional electrodes (for a total of 19 electrodes), it should be noted that more or fewer (including zero) additional electrodes may be included without departing from the spirit or scope of embodiments described herein and that the number and placement of the additional electrodes may be selected to optimize operation of the system 100 and increase a number of subsets to that may be evaluated for use, as will be described hereinbelow. Additionally and/or alternatively, placement of the additional electrodes may be influenced by characteristics of the human subject (e.g., phenotype) and/or suspected pathology, if that information is available. In certain embodiments, *M* is equal to three; however, it will be recognized that subsets including more or fewer leads may be employed without departing from the spirit or scope of embodiments described herein. It will further be recognized that steps performed by the system 100 as described below are implemented for each of *N* configurations of *M* leads.

As shown in FIG. 1A, during an initial training session performed by the system 100, electrodes comprising the enhanced ECG system 102 are placed on the torso and limbs of a human subject 104, with the electrodes comprising a 12-lead standard ECG system (e.g., electrode 103A) placed in the standard locations and each of the additional electrodes (e.g., electrode 103B) being placed in different locations. Signals acquired from the standard 12-lead system 106 and signals captured from an *M*-lead system or configuration 108 (comprising one of *N M-*lead systems or configurations), which may consist of three leads of the enhanced ECG system 102, are recorded and a portion (e.g., approximately 16 seconds in one embodiment) of each of the signals is input to a training module 110. In certain embodiments, signals from all electrodes of the enhanced ECG system 102 are recorded using a single device. In alternative embodiments, signals 106 may be captured using analog front ends ("AFEs") that provide clinical grade data (e.g., using commercially-available ECG carts) while signals 108 may be captured in parallel using one or more devices with non-clinical grade AFEs.

As will be described, the recorded signals may be used by the training module 110 to obtain coefficients of a trained model 112 that is used by a reconstruction module 114 to reconstruct the signals produced by the standard 12-lead system 106 from signals produced by the *M*-lead system 108. In particular, in the illustrated embodiment, the reconstruction module 114 may apply the remainder of the recorded signals from the 3-lead system 108 (e.g., approximately 1-2 minutes) to the trained model 112 and output reconstructed 12-lead ECG signals to an evaluation module 116. It will be recognized that training may be unnecessary once the system (and in particular, the model 112) proves robust (e.g., after a large number of cases have been analyzed), as the coefficients of the model may be inferred from previous information.

The evaluation module 116 checks the accuracy, reliability and/or trustworthiness of the reconstruction afforded using the *M-*lead system 108 with reference to the 12-lead ECG signals 106. For example, in certain embodiments, the evaluation module 116 may compare the reconstructed 12-lead ECG signals output from the reconstruction module 114 with the remainder of the original 12-lead ECG signals 106 captured by the standard 12-lead system using several Figures of Merit ("FoMs").

The FoMs calculated by the evaluation module 116 for each of the *N* configurations are input to a ranking module 118 which ranks the different configurations of the different configurations of the *M*-lead system 108 (which are recorded, used to train, and evaluated in the same manner described above with respect to the recorded standard 12-lead system signals 106) and outputs a personalized ranking of configurations/locations 120 for the subject 104. In one example embodiment, the ranking is performed using the FoMs for each of the *N M-*lead configurations; however, it will be recognized that any other number of methods of ranking, including methods which weight certain derivation characteristics or some FoMs more heavily than others (e.g., based on known pathologies or medical history of the subject), may be employed by the ranking module 118 without departing from the spirit or scope of embodiments described herein.

In certain embodiments, one or more of the *M*-lead combinations/configurations that produce the most (or one of the most) accurate, reliable, and/or trustworthy reconstructed 12-lead ECG signals and the model associated therewith are selected as the personalized system for the user, e.g., for at-home and/or ambulatory use.

As previously noted, in a particular embodiment, a first portion (e.g., 16 seconds) of each of the signals may be used by the training module 110 to train the model 112, which in certain embodiments comprises an ANN, while the remainder of each of the signals is used for reconstruction (reconstruction module 114), evaluation (evaluation module 116) , and ranking (ranking module 118). Additionally and/or alternatively, all of each of the signals may be used to perform the ranking. Additionally, in certain embodiments, the reliability, or trustworthiness, of the reconstruction associated with a particular *M*-lead combination may be validated based on known constraints, for example that the mathematical relationships between certain signals of the 12-lead standard system or certain relationships between the precordial leads are satisfied by the reconstruction.

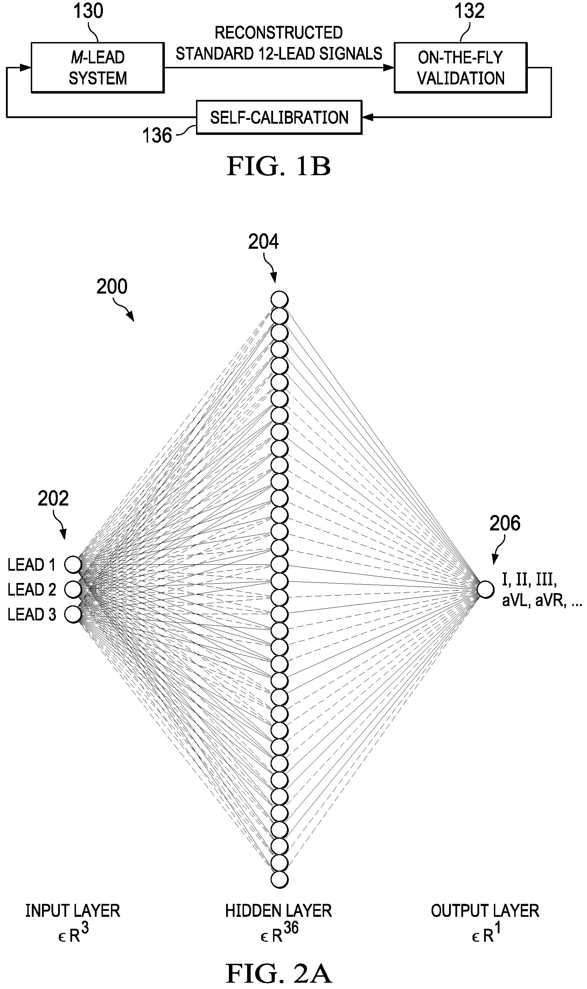

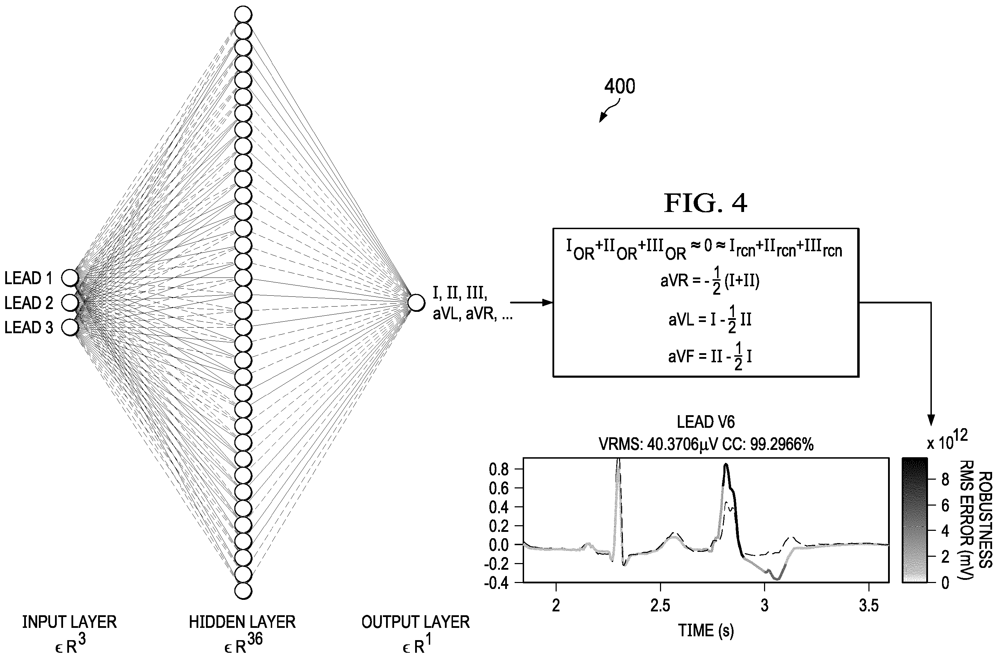

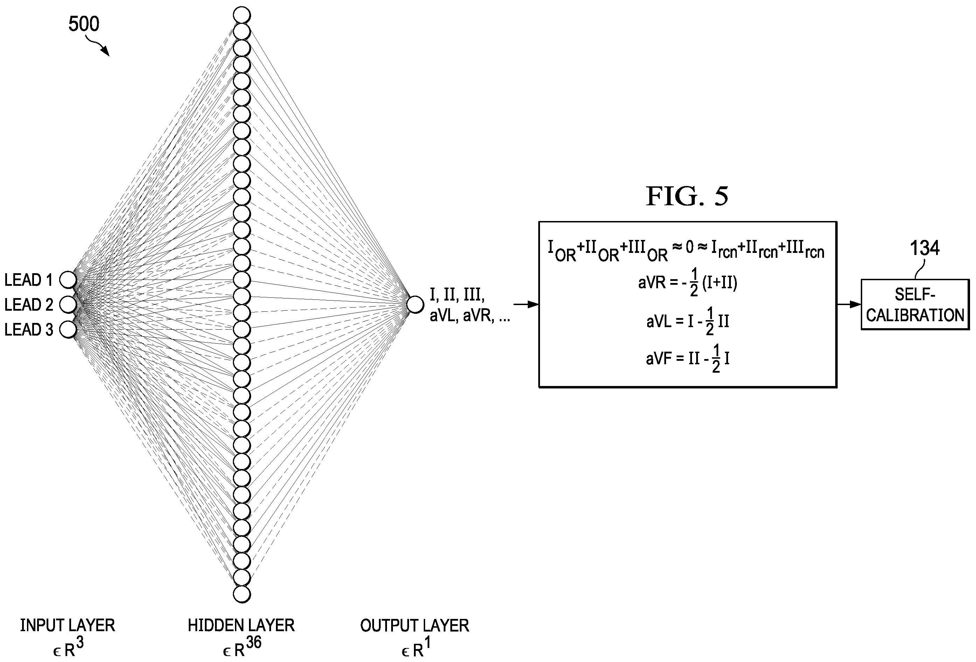

FIG. 1B is a block diagram illustrating an embodiment for on-the-fly evaluation, or assessment, and adaptation of an *M*-lead system 130, which may include, for example the one of the *M*-lead configurations selected from the personalized locations ranking 120 (FIG. 1A) and corresponding model, during use of the system 130 by a subject. As illustrated in FIG. 1B, the accuracy, reliability, and/or trustworthiness of reconstructed standard 12-lead ECG signals from the *M*-lead system 130 are assessed by an on-the-fly validation module 132 based on validating/evaluating the intrinsic relationships among some of the signals in the 12-lead standard ECG system (e.g., the limb leads and/or the precordial leads), as will be described. Additionally, as will be described in detail below (e.g., with reference to FIG. 5), as a result of the assessment, the on-the-fly validation module 132 may provide calibration data to a self-calibration module 134 for calibrating the *M*-lead system 130. Additionally and/or alternatively, as described in greater detail below, subsequent to the assessment by the on-the-fly validation module 132 (e.g., as illustrated in FIG. 4), a local confidence value may be assigned to the reconstruction based on the level of accuracy of the below-noted equations, which is indicative of the robustness of the reconstruction at a particular time or during a time window .

FIG. 2A illustrates an artificial neural network (ANN) 200 that may be used to implement the model for reconstructing a 12-lead standard ECG system using only three leads formed by four electrodes, such as illustrated in FIG. 1A. As shown in FIG. 2A, in one example embodiment, the ANN 200 is a single output ANN comprising a multilayer perceptron network (MLP) including an input layer 202 comprising three inputs respectively corresponding to the leads of a 3-lead system, a hidden layer 204, and an output layer comprising a single output corresponding to one of the 12 reconstructed leads of a 12-lead standard ECG system. It will be recognized that the model will include 12 single output ANNs 200, one for each of the 12 reconstructed leads.

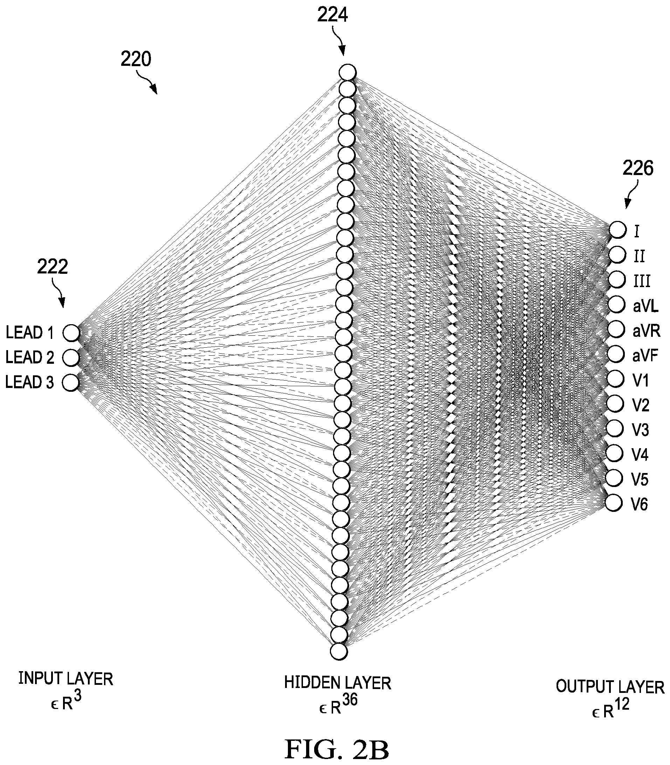

FIG. 2B illustrates an alternative embodiment of an ANN, designated in FIG. 2B by a reference numeral 220, that may be used to implement the model for reconstructing a 12-lead standard ECG system using only three leads formed by four electrodes, such as the system 100 (FIG. 1A). As shown in FIG. 2B, in one embodiment, the ANN 220 is a multiple output ANN comprising a multilayer perceptron network (MLP) including an input layer 222 comprising three inputs respectively corresponding to the leads of a 3-lead system, a hidden layer 224, and an output layer comprising multiple outputs each of which corresponds to one of the 12 reconstructed leads of a 12-lead standard ECG system. It will be recognized that either ANN arrangement 200, 220, or an alternative arrangement may be advantageously used to implement the embodiments as described herein.

It will be recognized that while single output ANNs, such as ANN 200, provide faster convergence, such that a system deploying single output ANNs will be faster to train, an advantage of using a multiple output ANN, such as ANN 220, is that deviations in mathematical relations among the outputs could be fed back to the ANN to update the weights. Additionally, although as illustrated and described herein, the ANN 200 includes a single hidden layer 204, the number of neurons in the hidden layer should not be read as being restricted or limited to a particular number. Similarly, although as illustrated and described herein, the ANN 220 includes a single hidden layer 224, the number of hidden layers should not be read as being restricted or limited to a particular number. Moreover, although embodiments described herein consider the acquired leads, additional inputs related to the acquired leads (e.g., angles and magnitude of the cardiac vector) and/or the subject (e.g., gender, age, know pathology and/or comorbidities) may be implemented/added as inputs to the ANN (e.g., ANN 200, 220) as desired in order to accelerate the convergence of the network.

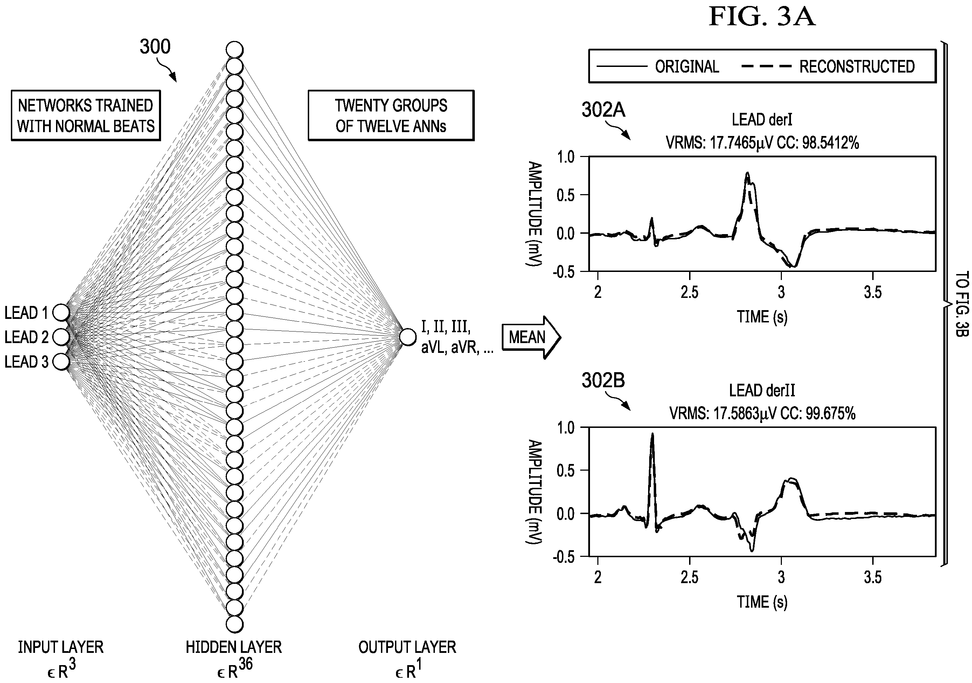

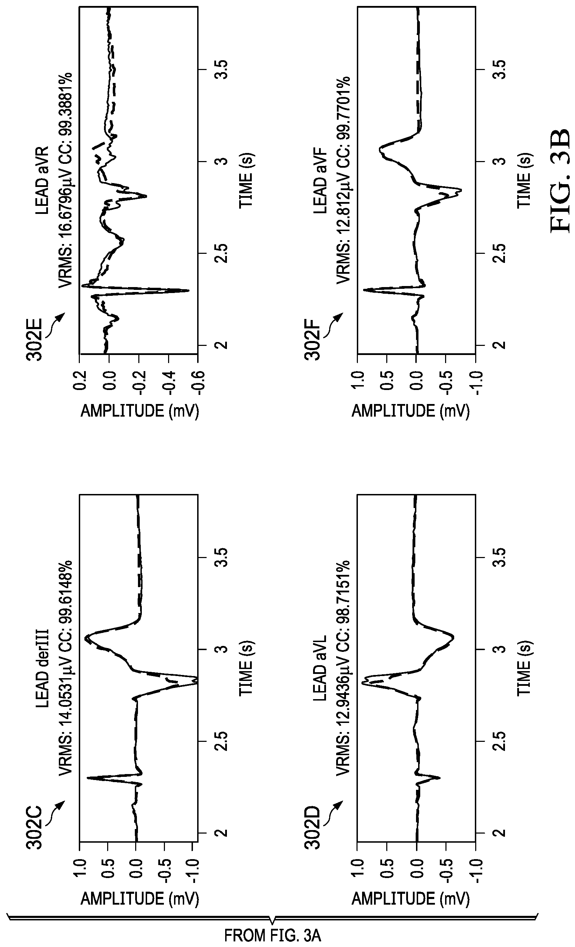

Expert committees comprising different regressors may be implemented in order to increase the robustness of the regression. FIGs. 3A-3B illustrate a manner in which the regressor's output against previously unknown or untrained events (such as ectopic heartbeat or pathology) may be improved using expert committees. In one embodiment such a committee of experts is implemented as a group of ANNs (instead of a single ANN) for reconstructing each lead of the 12-lead standard ECG system from the *M-*lead system. In the embodiment shown in FIGs. 3A-3B, an expert committee considering 20 runs of the regressor that involves one ANN per lead, 12 in total, is deployed, with the mean value for each lead being used to reconstruct the lead signal. In one example, as illustrated in FIG. 3A, a reconstruction algorithm including a network 300 comprising expert committees trained using only normal heartbeats may be used to successfully reconstruct leads comprising normal beats plus premature ventricular contractions (PVCs), represented by graphs 302A-302F. It will be recognized that other pathologies may be successfully reconstructed using a system trained using only normal heartbeats.

It will also be recognized that, although FIG. 3A illustrates use of 20 runs (20 ANNs in this case), more or fewer ANNs may be deployed. Graphs 302A-302F illustrate an original lead signal versus the reconstructed lead signal for leads I, II, III, aVL, aVR, and aVF, respectively. Additionally and/or alternatively, different and/or additional regressors (e.g., linear regression, CNN, binary trees) may be used to increase robustness of the committee (or final regressor).

FIG. 4 illustrates a system 400 in which the accuracy, reliability, and/or trustworthiness, of the reconstruction of leads can be evaluated or assessed based on the intrinsic characteristics of the relationship among some of the signals in the 12-lead standard ECG system. As represented in FIG. 4, system 400, all or a portion of which may be used to implement on-the-fly validation module 132 (FIG. 1B), may analyze the following four equations: $I - II + III \approx 0$ $aVR = - \frac{1}{2} \left(I+II\right)$ $aVL = I - \frac{1}{2} II$ $aVF = II - \frac{1}{2} I$ and, based on the accuracy of the results, determines the robustness (or reliability) of the reconstruction for the leads. This is performed without access to the actual 12-lead standard ECG derivations as used during the initial training phase (FIG. 1A) and enables continuous validation on the fly (e.g., while the system is deployed on a subject) or offline when the data is processed, as well as dynamic retraining of the system.

As previously noted, subsequent to the assessment, a local confidence value may be assigned to the reconstruction based on the level of accuracy of the above-noted equations, which is indicative of the robustness of the reconstruction. In one embodiment, the confidence value may be normalized to a value between a first value (e.g., 0) indicating that the reconstruction is highly untrustworthy, and a second value (e.g., 1) indicating that the reconstruction is highly trustworthy; a value between the first and second value indicates a relative trustworthiness/untrustworthiness of the reconstruction. In another embodiment, the confidence value may comprise one of a number of values, with each value indicating a relative trustworthiness and/or acceptability of the reconstruction. Additionally and/or alternatively, the trustworthiness of the acquired signals may be considered in assessing the reconstructed signals and assigning a confidence value. For example, if the acquired signals are very noisy, the confidence level of the reconstruction may be lower than if the acquired signals are less noisy. The same may be true for situations in which heavy motion is detected (e.g., using an accelerometer), in which case signals acquired under conditions of high motion may result in the reconstruction being deemed less trustworthy than would be the case for a reconstruction performed using signals acquired under more static conditions. In certain embodiments, precordial leads may also be evaluated to increase trust on the assessment of the reconstructed signals. It will be recognized that this evaluation (or assessment) may be performed throughout operation of the system described herein. Information from ancillary sensors could be also considered, as in the case of an accelerometer that may detect a heavy motion or aggressive exercise that could degrade/compromise reconstruction.

FIG. 5 illustrates a system 500 in which the regressor's weights may be adapted depending on the reconstruction quality based on the intrinsic relationship among the different signals in the 12-lead standard system. All or a portion of the system 500 may be used to implement the on-the-fly validation module 132 (FIG. 1B) and self-calibration module 134 (FIG. 1A). This adaptation, or self-calibration, may be triggered in response to a reconstruction being assigned a confidence value below a certain threshold. Additionally, the confidence value may be directly used in the adaptation of the weights/self-calibration process. The system is allowed to continue adapting its weights on the run where the deviation is used as an error signal. In certain embodiments, the weight adaptation is limited to avoid a global false minimum, as when all outputs are zeroed.

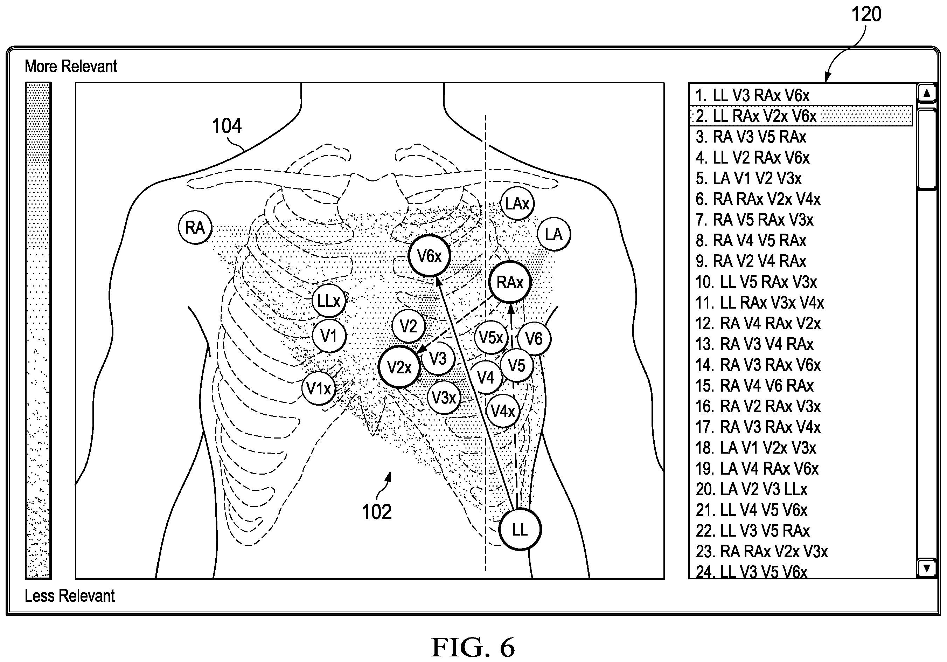

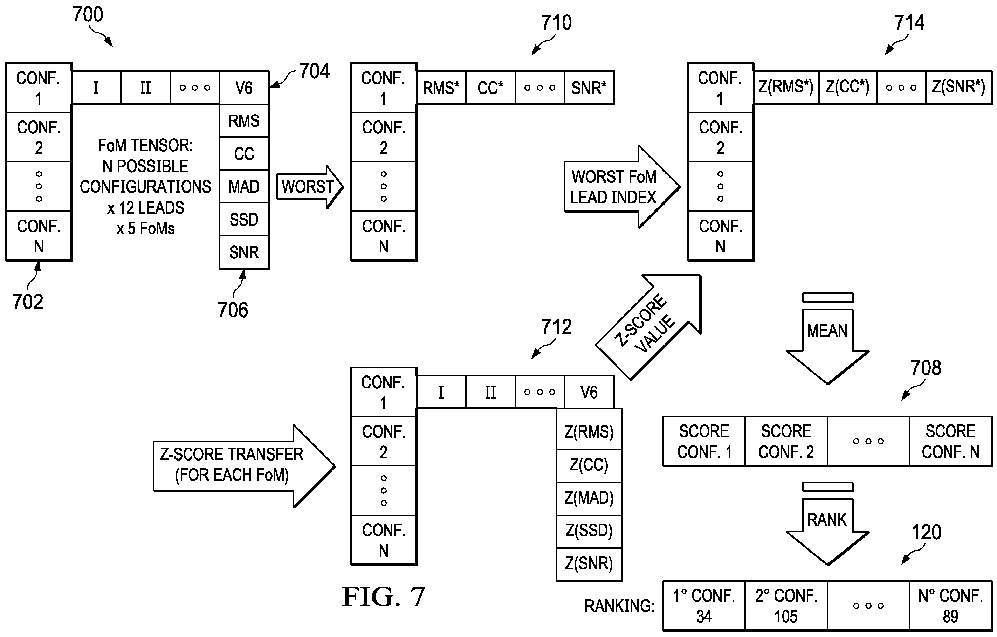

FIG. 6 illustrates a manner in which the personalized locations ranking 120 of a number (e.g., *N*) of *M-*lead combinations/configurations (which in the illustrated embodiment comprise 3-lead configurations) specific to each subject may be generated by the ranking module 118 (FIG. 1A) based on the mathematical accuracy of the various configurations to ensure the best possible signal reconstruction for the particular subject. Subsequent to the mathematical ranking, heuristics may be applied to alter the order and/or to determine which of the configurations should be used based on various restrictions and other considerations. Such restrictions may be defined by physiological or anatomical constraints and practical effect (e.g., in order to adapt to the anatomy of the subject and/or to limit distance between electrodes), as well as the form factor of the device. In particular, FIG. 6 depicts the ranked list 120 of a portion of the possible 3-lead system configurations/combinations ranked in order of a configuration score (FIG. 7) for the human subject 104. Since the degradation of performance is relatively smooth (i.e., configurations separated 10 positions in the ranked list 120 typically have nearly equal performance), the list 120 may be used to select one or more 3-lead combinations/configurations to be used by the subject 104, e.g., in an ambulatory device, based on anatomical restrictions of the subject 104 (e.g., breast tissue) and/or clinical considerations (e.g., a particular feature of the ECG is of interest and a given direction is known to have more information than others). In one embodiment, the ability to select more than one similarly performing (i.e., similarly ranked) configuration of *M* leads enables the subject to alternate placement of the electrodes over time, thereby potentially preventing irritation or damage to the subject's skin.

FIG. 7 illustrates an example ranking algorithm for implementation by the ranking module 118 (FIG. 1A) for ranking *M*-lead system configurations in accordance with embodiments described herein. As previously noted, there are many different manners in which to rank the system configurations; the ranking method illustrated in FIG. 7 is merely an example of how such a ranking may be accomplished. As shown in FIG. 7, in one example embodiment, an FoM tensor 700 is defined including *N* possible electrode configurations times 12 leads times *Y* FoMs, for the current example. In certain embodiments, *Y* is equal to five; however, it will be recognized that more or fewer than five FoMs may be defined. In one example embodiment, the FoMs considered may include root mean squared (RMS), cross-correlation (CC), maximum absolute distance (MAD), sum of squared distance (SSD) and signal-to-noise ratio (SNR). In accordance with features of example embodiments described herein, a unique score 708 is derived for each configuration by extracting the worst FoM for all leads in the configuration 710, determining a Z-score (i.e., the distance from the mean in standard deviations) for each of the FoMs 712 and combining the Z-scores into a single value 714, which is the configuration score for the configuration. The configurations are then ranked by configuration score to determine which configuration provides the best reconstruction for the particular subject to generate the ranked list 120.

It will be recognized that there will be several configurations that may provide acceptable reconstructions and that, as discussed above, in addition to rank, other factors, such as physiological constraints, practical effects, and/or pathology being studied, for example, may also impact which configuration is ultimately selected as optimal for a particular application.

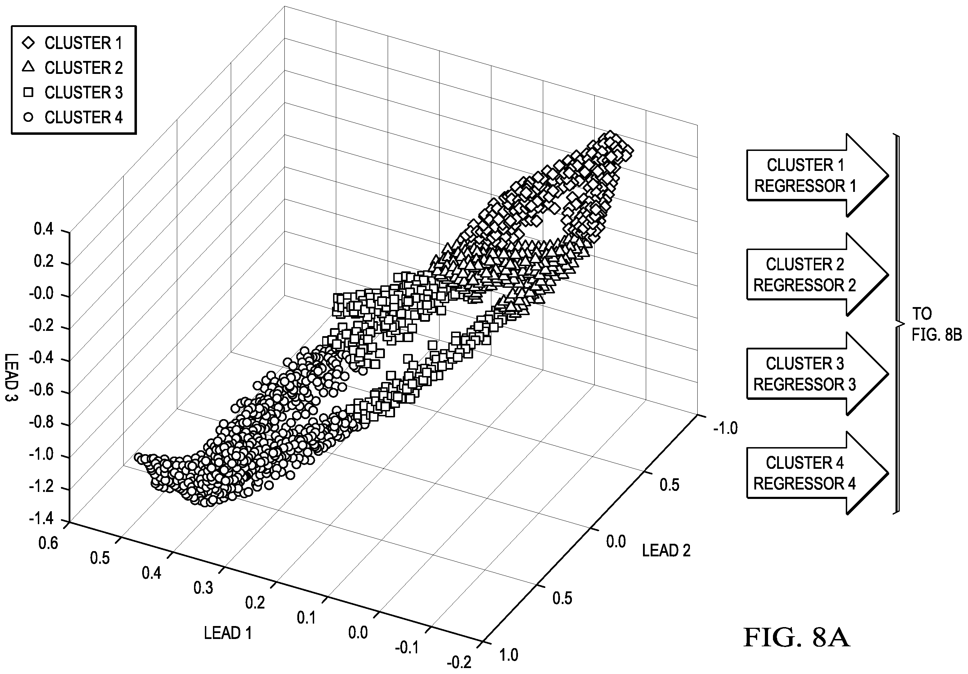

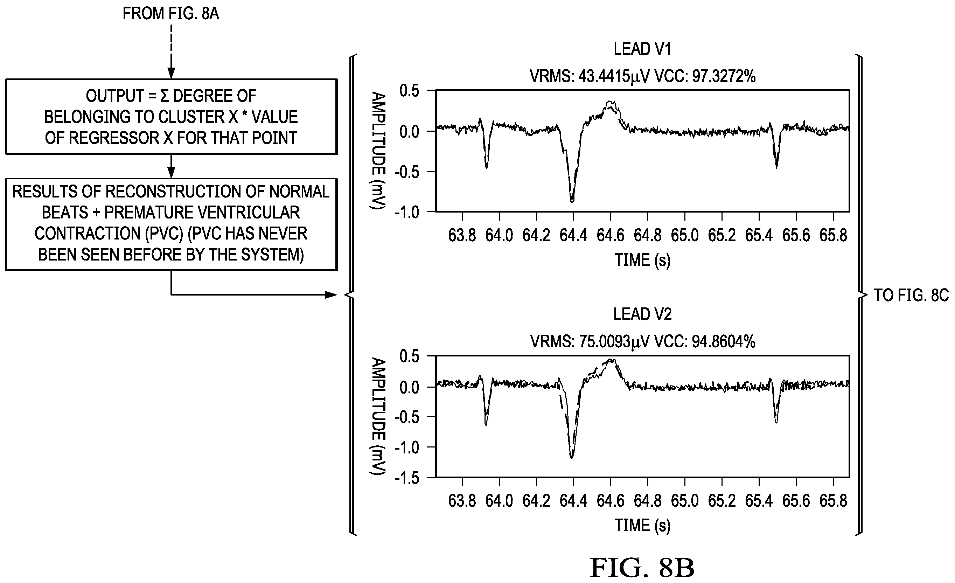

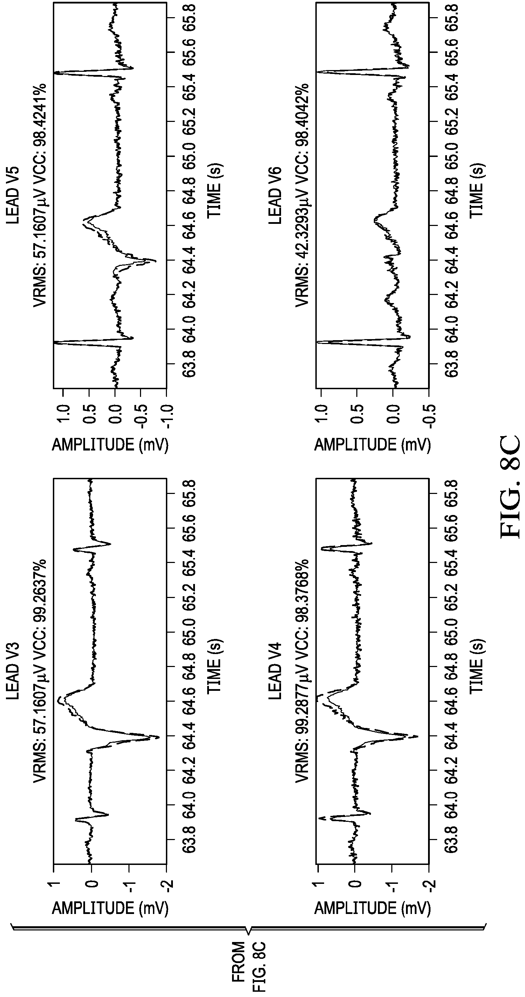

FIGs. 8A-8C collectively illustrate an alternative embodiment of a reconstruction algorithm that is based on *a priori* clustering of input data using fuzzy c-means (FCM). As shown in FIGs. 8A-8C, partitioning, or clustering, of the input data may be performed "automatically" by the reconstruction algorithm during the training phase through optimization processes based on machine learning principles. After the data is partitioned, specific regressors may be applied to each cluster, trained specifically for each of its sets. The use of fuzzy clusters instead of classic clusters enables a smoother transition, assigning principles of membership instead of static and/or exclusive labels.

In other words, each data sample is assigned a degree of belonging to each cluster based on how well the cluster represents the sample. For example, a data sample may have a 30% degree of belonging to a cluster A, a 20% degree of belonging to a cluster B, a 50% degree of belonging to a cluster C, and a 0% degree of belonging to a cluster D. The missing leads are reconstructed by combining the individual regressors with that same weight. In an example embodiment, C-means is applied for clustering and a specific linear regressor is applied for each cluster. Once the leads are reconstructed, a determination is made whether the known relations among derivations (e.g., III = II - I, aVL = ½ (I- III) AVR= -½ (I + II) aVF=1/2 (II + III)) are met. This determination is used to assign a confidence level that defines the robustness and/or reliability of the reconstruction, as described above with reference to the ANN embodiments.

In an illustrative embodiment, four models may be built to describe the repolarization and depolarization of the atria and ventricles of a heart separately; however, since there are areas in which they coexist, the system may provide for a combination of the models in certain areas of the beat type of chambers are activated. In one embodiment, the approach is not based on an aprioristic model but on a statistical analysis.

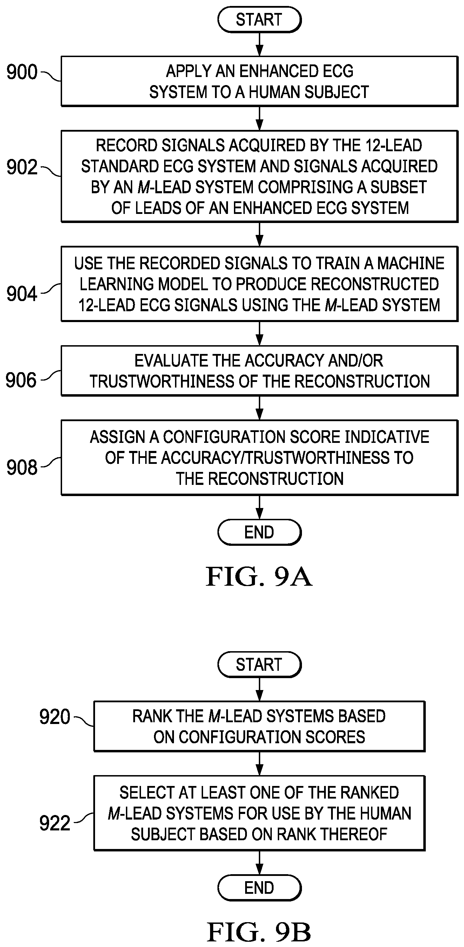

FIGs. 9A and 9B are flow diagrams illustrating operation of a technique for ECG lead reconstruction in accordance with features of embodiments described herein, which may be implemented by system 100 (FIG. 1A). Referring to FIG. 9A, in step 900, an ECG system is applied to a human subject. For example, in the embodiment illustrated in FIG. 1A, an enhanced ECG system including 19 electrodes comprising electrodes of a standard 12-lead ECG system as well as nine additional electrodes are applied to the subject's limbs and torso in accordance with embodiments described herein.

In step 902, signals acquired by the 12-lead standard ECG system and signals acquired by a selected *M*-lead ECG system comprising a subset of leads of the enhanced ECG system are recorded.

In step 904, some portion of the recorded signals are used to train a machine learning model to produce reconstructed 12-lead signals using the *M* lead system. In one embodiment, the first approximately 16 seconds of the recorded signals are used to train the machine learning model in this manner.

In step 906, the accuracy, reliability and/or trustworthiness of the reconstruction may be evaluated with reference to the signals acquired by the 12-lead standard ECG system.

In step 908, a configuration score indicative of the evaluated accuracy, reliability and/or trustworthiness of the reconstruction may be assigned to the *M-*lead ECG system. In certain embodiments, the configuration score may be assigned with reference to FoMs of the reconstruction.

It will be recognized that the steps illustrated in FIG. 9A may be performed for each of *N M-*lead configurations comprising a subset of leads of the enhanced ECG system such that each *M*-lead configuration is assigned a configuration score indicative of the accuracy, reliability and/or trustworthiness of the reconstruction enabled by the configuration as applied to the particular human subject.

Referring now to FIG. 9B, in step 920, all *N* of the *M*-lead configurations are ranked based on a configuration score assigned to the configuration (e.g., based on FoMs and/or some other ranking method).

In step 922, at least one of the *N M-*lead configurations is selected for use in connection with the human subject based on a rank of the selected system. For example, the highest ranking or one of the higher ranking configurations may be selected. The selected *M-*lead configuration may be deployed in form factor for an ambulatory device for use by the human subject. As noted above, in certain embodiments, more than one similarly ranked configuration of *M* leads may be identified for enabling the subject to alternate placement of the electrodes over time, thereby to reduce the possibility of irritation or damage to the subject's skin.

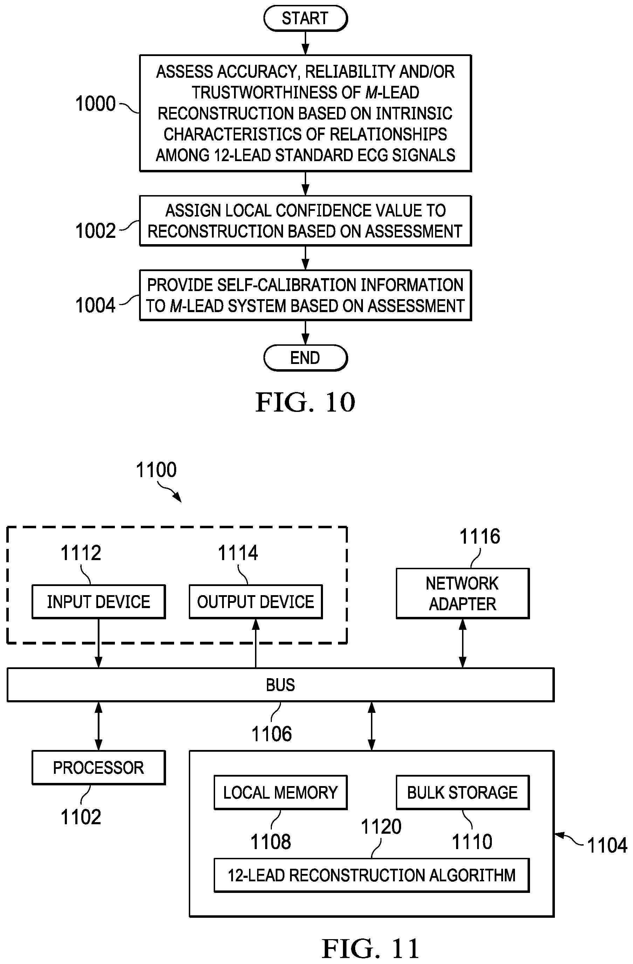

FIG. 10 is a flow diagram illustrating a technique for on-the-fly analysis and potential adaptation of an ECG lead reconstruction system in accordance with features of embodiments described herein (e.g., as illustrated in FIG. 1B). In step 1000, the accuracy, reliability and/or trustworthiness of the reconstruction provided by an *M*-lead ECG reconstruction system may be assessed, or validated, on-the-fly based on the intrinsic characteristics of the relationship among some of the signals in a 12-lead standard ECG system (as described with reference to FIG. 4), relations between precordial derivations or information of additional sensors, such as accelerometers or estimations of contact impedance of the electrodes.

In step 1002, a local confidence value indicative of the assessed accuracy/reliability and/or trustworthiness of the reconstruction may be assigned to the *M*-lead ECG reconstruction system. The confidence value may be used to determine a level of confidence that may be placed in the reconstruction at a particular instant or window in time.

In step 1004, results of the assessment performed in step 1000 may be used by the *M-*lead ECG reconstruction system to perform self-calibration (as described with reference to FIG. 5), thereby to improve operation thereof.

It will be recognized that the steps illustrated in FIG. 10 may be performed "-on-the-fly" and the assigned confidence value may be associated with a point or window in time of a reconstructed signal and may therefore change over time, as noted above. It will also be recognized that the steps illustrated in FIG. 10 are optional and independent and may be omitted in connection with systems in which the accuracy, reliability and/or trustworthiness thereof have already been established.

FIG. 11 is a block diagram illustrating an example system 1100 that may be configured to implement at least portions of a system for ECG lead reconstruction in accordance with features of embodiments described herein, and more particularly as shown in the FIGURES described hereinabove. As shown in FIG. 11, the system 1100 may include at least one processor 1102, e.g. a hardware processor 1102, coupled to memory elements 1104 through a system bus 1106. As such, the system may store program code and/or data within memory elements 1104. Further, the processor 1102 may execute the program code accessed from the memory elements 1104 via a system bus 1106. In one aspect, the system may be implemented as a computer that is suitable for storing and/or executing program code. It should be appreciated, however, that the system 1100 may be implemented in the form of any system including a processor and a memory that is capable of performing the functions described in this disclosure.

In some embodiments, the processor 1102 can execute software or an algorithm to perform the activities as discussed in this specification; in particular, activities related to ECG lead reconstruction in accordance with features of embodiments described herein. The processor 1102 may include any combination of hardware, software, or firmware providing programmable logic, including by way of non-limiting example a microprocessor, a DSP, a field-programmable gate array (FPGA), a programmable logic array (PLA), an integrated circuit (IC), an application specific IC (ASIC), or a virtual machine processor. The processor 1102 may comprise a cloud processor. The processor 1102 may be communicatively coupled to the memory element 1104, for example in a direct-memory access (DMA) configuration, so that the processor 1102 may read from or write to the memory elements 1104.

In general, the memory elements 1104 may include any suitable volatile or nonvolatile memory technology, including double data rate (DDR) random access memory (RAM), synchronous RAM (SRAM), dynamic RAM (DRAM), flash, read-only memory (ROM), optical media, virtual memory regions, magnetic or tape memory, or any other suitable technology. Unless specified otherwise, any of the memory elements discussed herein should be construed as being encompassed within the broad term "memory." The information being measured, processed, tracked, or sent to or from any of the components of the system 1100 could be provided in any database, register, control list, cache, or storage structure, all of which can be referenced at any suitable timeframe. Any such storage options may be included within the broad term "memory" as used herein. Similarly, any of the potential processing elements, modules, and machines described herein should be construed as being encompassed within the broad term "processor." Each of the elements shown in the present figures may also include suitable interfaces for receiving, transmitting, and/or otherwise communicating data or information in a network environment so that they can communicate with, for example, a system having hardware similar or identical to another one of these elements.

In certain example implementations, mechanisms for implementing a system for ECG lead reconstruction as outlined herein may be implemented by logic encoded in one or more tangible media, which may be inclusive of non-transitory media, e.g., embedded logic provided in an ASIC, in DSP instructions, software (potentially inclusive of object code and source code) to be executed by a processor, or other similar machine, etc. In some of these instances, memory elements, such as e.g. the memory elements 1104 shown in FIG. 11, can store data or information used for the operations described herein. This includes the memory elements being able to store software, logic, code, or processor instructions that are executed to carry out the activities described herein. A processor can execute any type of instructions associated with the data or information to achieve the operations detailed herein. In one example, the processors, such as e.g. the processor 1102 shown in FIG. 11, could transform an element or an article (e.g., data) from one state or thing to another state or thing. In another example, the activities outlined herein may be implemented with fixed logic or programmable logic (e.g., software/computer instructions executed by a processor) and the elements identified herein could be some type of a programmable processor, programmable digital logic (e.g., an FPGA, a DSP, an erasable programmable read-only memory (EPROM), an electrically erasable programmable read-only memory (EEPROM)) or an ASIC that includes digital logic, software, code, electronic instructions, or any suitable combination thereof.

The memory elements 1104 may include one or more physical memory devices such as, for example, local memory 1108 and one or more bulk storage devices 1110. The local memory may refer to RAM or other non-persistent memory device(s) generally used during actual execution of the program code. A bulk storage device may be implemented as a hard drive or other persistent data storage device. The processing system 1100 may also include one or more cache memories (not shown) that provide temporary storage of at least some program code in order to reduce the number of times program code must be retrieved from the bulk storage device 1110 during execution.

As shown in FIG. 11, the memory elements 1104 may store an ECG lead reconstruction module 1120. In various embodiments, the module 1120 may be stored in the local memory 1108, the one or more bulk storage devices 1110, or apart from the local memory and the bulk storage devices. It should be appreciated that the system 1100 may further execute an operating system (not shown in FIG. 11) that can facilitate execution of the module 1120. The module 1120, being implemented in the form of executable program code and/or data, can be read from, written to, and/or executed by the system 1100, e.g.., by the processor 1102. Responsive to reading from, writing to, and/or executing the module 1120, the system 1100 may be configured to perform one or more operations or method steps described herein, such as shown in and described with reference to FIGs. 9 and 10.

Input/output (I/O) devices depicted as an input device 1112 and an output device 1114, optionally, may be coupled to the system. Examples of input devices may include, but are not limited to, a keyboard, a pointing device such as a mouse, or the like. Examples of output devices may include, but are not limited to, a monitor or a display, speakers, or the like. In some implementations, the system may include a device driver (not shown) for the output device 1114. Input and/or output devices 1112, 1114 may be coupled to the system 1100 either directly or through intervening I/O controllers.

In an embodiment, the input and the output devices may be implemented as a combined input/output device (illustrated in FIG. 11 with a dashed line surrounding the input device 1112 and the output device 1114). An example of such a combined device is a touch sensitive display, also sometimes referred to as a "touch screen display" or simply "touch screen". In such an embodiment, input to the device may be provided by a movement of a physical object, such as e.g. a stylus or a finger of a user, on or near the touch screen display.

A network adapter 1116 may also, optionally, be coupled to the system 1100 to enable it to become coupled to other systems, computer systems, remote network devices, and/or remote storage devices through intervening private or public networks. The network adapter may comprise a data receiver for receiving data that is transmitted by said systems, devices and/or networks to the system 1100, and a data transmitter for transmitting data from the system 1100 to said systems, devices and/or networks. Modems, cable modems, and Ethernet cards are examples of different types of network adapter that may be used with the system 1100.

Example 1 is a method for reconstructing 12-lead standard electrocardiogram (ECG) system signals for a human subject using an *M* lead system, the method including recording signals acquired by a 12-lead standard ECG system; recording signals acquired by the *M*-lead system; and using the recorded signals to train a machine learning model to produce the reconstructed 12-lead standard ECG system signals using the *M*-lead system.

In Example 2, the method of Example 1 may further include the *M*-lead system including *M* leads comprising a subset of leads of an enhanced ECG system and wherein the enhanced ECG system includes the 12-lead standard ECG system.

In Example 3, the method of Example 2 may further include the enhanced ECG system including at least one additional electrode.

In Example 4, the method of any of Examples 1-3 may further include evaluating a performance of the machine learning model by comparing the recorded 12-lead standard EDG system signals with the reconstructed 12-lead standard ECG system signals.

In Example 5, the method of any of Examples 1-4 may further include the machine learning model comprising an artificial neural network (ANN), wherein a portion of each of the recorded signals is used to train coefficients of the ANN.

In Example 6, the method of Example 5 may further include the ANN comprising a multiple output ANN.

In Example 7, the method of Example 5 may further include the ANN comprising multiple single output ANNs.

In Example 8, the method of Example 5 may further include the ANN comprising *M* inputs corresponding to leads of the *M*-lead system.

In Example 9, the method of Example 8 may further include the ANN comprising at least one additional input corresponding to at least one of an angle of a cardiac vector, a magnitude of the cardiac vector, and information regarding the human subject.

In Example 10, the method of any of Examples 1-9 may further include the machine learning model comprising a committee of experts.

In Example 11, the method of any of Examples 1-10 may further include the *M-*lead system comprising a plurality of *M*-lead systems, the method further including evaluating an accuracy of each of the *M*-lead systems; and ranking the *M*-lead systems in order of the accuracy thereof.

In Example 12, the method of Example 11 may further include the evaluating an accuracy of each of the *M*-lead systems being performed with reference to *Y* figures of merit (FoMs) of each of the *M*-lead systems.

In Example 13, the method of Example 11 may further include selecting one of the *M*-lead systems for use in monitoring an ECG of the human subject based on the ranking of the selected one of the *M*-lead systems.

In Example 14, the method of Example 11 may further include selecting multiple ones of the *M*-lead systems for use in monitoring an ECG of the human subject based on the rankings of the selected multiple ones of the *M*-lead systems.

In Example 15, the method of Example 13 may further include assessing an accuracy of a reconstruction produced by the selected one of the *M*-lead systems by determining whether intrinsic characteristics of standard 12-lead ECG signals are met by the reconstruction and assigning a confidence value to the reconstruction based on results of the assessing.

In Example 16, the method of Example 15 may further include performing calibration of the selected one of the *M*-lead systems based on the results of the assessing.

In Example 17, the method of Example 13 may further include assessing a trustworthiness of a reconstruction produced by the selected one of the *M-*lead systems by based on at least one of external sensor data and contact impedance data.

In Example 18, the method of any of Examples 1-17 may further include the machine learning model being implemented using fuzzy c-means (FCM) with regressors.

In Example 19, the method of any of Examples 1-18 may further include *M* being equal to 3.

Example 20 is an electrocardiogram (ECG) reconstruction system for reconstructing 12-lead standard ECG system signals using an *M*-lead system, the ECG reconstruction system comprising a plurality of electrodes comprising the 12-lead standard ECG system, wherein the plurality of electrodes are applied to skin of a human subject; a training module for using signals acquired by the 12-lead standard ECG system and signals acquired by the *M*-lead system to train a machine learning model to reconstruct 12-lead standard ECG system signals from the signals acquired by the *M*-lead system; and a reconstruction module for using the machine learning model to reconstruct the 12-lead standard ECG system signals using the *M-*lead system.

In Example 21, the ECG reconstruction system of Example 20 may further include the *M*-lead system comprising *M* leads of an enhanced ECG system that includes the 12-lead standard ECG system.

In Example 22, the ECG reconstruction system of Example 21 may further include the enhanced ECG system comprising at least one additional electrode.

In Example 23, the ECG reconstruction system of any of Examples 20-23 may further include an evaluation module for evaluating an accuracy of the machine learning model.

In Example 24, the ECG reconstruction system of claim 23 may further include the evaluation module evaluating the accuracy of the machine learning model by comparing the signals acquired by the 12-lead standard ECG system with the reconstructed signals.

In Example 25, the ECG reconstruction system of any of Examples 20-24 may further include the machine learning model comprising an artificial neural network (ANN) and wherein a portion of each of the recorded signals is used to train coefficients of the ANN.

In Example 26, the ECG reconstruction system of Example 25 may further include the ANN comprising a multiple output ANN.

In Example 27, the ECG reconstruction system of Example 25 may further include the ANN comprising multiple single output ANNs.

In Example 28, the ECG reconstruction system of any of Examples 20-27 may further include the machine learning model being implemented using committees of experts.

In Example 29, the ECG reconstruction system of any of Examples 20-28 may further include the machine learning model being implemented using fuzzy c-means (FCM) with regressors.

In Example 30, the ECG reconstruction system of any of Examples 20-28 may further include the *M*-lead system comprising multiple *M*-lead systems and wherein the evaluating module further evaluates an accuracy of each of the *M*-lead systems, the reconstruction system further comprising a ranking module for ranking the *M*-lead systems based on the accuracy of each of the *M*-lead systems.

In Example 31, the ECG reconstruction system of Example 30 may further include the evaluating an accuracy of each of the *M*-lead systems being performed with reference to *Y* figures of merit (FoMs) of each of the *M*-lead systems.

In Example 32, the ECG reconstruction system of Example 30 may further include an assessment module for assessing whether intrinsic characteristics of the standard 12-lead ECG signals are met by a reconstruction produced by a selected one of the multiple *M*-lead systems and assigning a confidence value to the machine learning model based on results of the assessing.

In Example 33, the ECG reconstruction system of Example 32 may further include a calibration module for calibrating the selected one of the multiple *M*-lead models based on the results of the assessing.

In Example 34, the ECG reconstruction system of any of Examples 20-33 may further include *M* being equal to 3.

Example 35 is a method for reconstructing 12-lead standard electrocardiogram (ECG) system signals using an *M* lead system, the method comprising recording first signals produced by the 12-lead standard ECG system; recording second signals produced by the set of *M* leads; training a machine learning model using a first portion of the first recorded signals and the second recorded signals; producing reconstructed signals by applying the machine learning model to a second portion of the second recorded signals; and evaluating an accuracy of the machine learning model by comparing the first signals with the reconstructed signals.

In Example 36, the method of Example 35 may further include the machine learning model comprising an artificial neural network (ANN) and wherein a portion of each of the recorded signals is used to train coefficients of the ANN.

In Example 37, the method of Example 36 may further include the ANN comprising a multiple output ANN.

In Example 38, the method of Example 36 may further include the ANN comprising a multiple single-output ANNs.

In Example 39, the method of any of Examples 35-38 may further include the machine learning model comprising a committee of experts.

In Example 40, the method of any of Examples 35-39 may further include assessing an accuracy of the machine learning model by determining whether intrinsic characteristics of the first signals are met by the reconstructed signals and assigning a confidence value to the machine learning model based on results of the assessing.

In Example 41, the method of Example 40 may further include adjusting weights of regressors of the machine learning model based on the confidence value.

In Example 42, the method of any of Examples 35-41 may further include the *M-*lead system comprising multiple *M*-lead systems, the method further comprising evaluating an accuracy of each of the *M*-lead systems; and ranking the unique sets based on the accuracy of each of the *M*-lead systems relative to the others.

In Example 43, the method of Example 42 may further include the evaluating an accuracy of each of the *M*-lead systems being performed with reference to *Y* figures of merit (FoMs) of the *M*-lead system.

In Example 44, the method of any of Examples 42-43 may further include selecting one of the *M*-lead systems for use in monitoring an ECG of the human subject based on a ranking of the selected one of the *M*-lead systems.

In Example 45, the method of Example 44 may further include assessing an accuracy of a reconstruction produced by the selected one of the *M*-lead systems by determining whether intrinsic characteristics of standard 12-lead ECG signals are met by the reconstruction and assigning a confidence value to the reconstruction based on results of the assessing.

In Example 46, the method of Example 45 may further include performing calibration of the selected one of the *M*-lead systems based on the results of the assessing.

In Example 47, the method of any of Examples 42-43 may further include selecting multiple ones of the *M*-lead systems for use in monitoring an ECG of the human subject based on rankings of the selected ones of the *M*-lead systems.

In Example 48, the method of any of Examples 35-47 may further include the machine learning model being implemented using fuzzy c-means (FCM) with regressors.

In Example 49, the method of any of Examples 35-48 may further include *M* equal to 3.

It should be noted that all of the specifications, dimensions, and relationships outlined herein (e.g., the number of elements, operations, steps, etc.) have only been offered for purposes of example and teaching only. Such information may be varied considerably without departing from the spirit of the present disclosure, or the scope of the appended claims. The specifications apply only to one non-limiting example and, accordingly, they should be construed as such. In the foregoing description, exemplary embodiments have been described with reference to particular component arrangements. Various modifications and changes may be made to such embodiments without departing from the scope of the appended claims. The description and drawings are, accordingly, to be regarded in an illustrative rather than in a restrictive sense.

Note that with the numerous examples provided herein, interaction may be described in terms of two, three, four, or more electrical components and/or modules. However, this has been done for purposes of clarity and example only. It should be appreciated that the system may be consolidated in any suitable manner. Along similar design alternatives, any of the illustrated components, modules, and elements of the FIGURES may be combined in various possible configurations, all of which are clearly within the broad scope of this Specification. In certain cases, it may be easier to describe one or more of the functionalities of a given set of flows by only referencing a limited number of electrical elements. It should be appreciated that the electrical circuits of the FIGURES and its teachings are readily scalable and may accommodate a large number of components, as well as more complicated/sophisticated arrangements and configurations. Accordingly, the examples provided should not limit the scope or inhibit the broad teachings of the electrical circuits as potentially applied to myriad other architectures.

It should also be noted that in this Specification, references to various features (e.g., elements, structures, modules, components, steps, operations, characteristics, etc.) included in "one embodiment", "exemplary embodiment", "an embodiment", "another embodiment", "some embodiments", "various embodiments", "other embodiments", "alternative embodiment", and the like are intended to mean that any such features are included in one or more embodiments of the present disclosure, but may or may not necessarily be combined in the same embodiments.

It should also be noted that the functions related to circuit architectures illustrate only some of the possible circuit architecture functions that may be executed by, or within, systems illustrated in the FIGURES. Some of these operations may be deleted or removed where appropriate, or these operations may be modified or changed considerably without departing from the scope of the present disclosure. In addition, the timing of these operations may be altered considerably. The preceding operational flows have been offered for purposes of example and discussion. Substantial flexibility is provided by embodiments described herein in that any suitable arrangements, chronologies, configurations, and timing mechanisms may be provided without departing from the teachings of the present disclosure.

Numerous other changes, substitutions, variations, alterations, and modifications may be ascertained to one skilled in the art and it is intended that the present disclosure encompass all such changes, substitutions, variations, alterations, and modifications as falling within the scope of the appended claims.

Note that all optional features of the device and system described above may also be implemented with respect to the method or process described herein and specifics in the examples may be used anywhere in one or more embodiments.

The 'means for' in these instances (above) may include (but is not limited to) using any suitable component discussed herein, along with any suitable software, circuitry, hub, computer code, logic, algorithms, hardware, controller, interface, link, bus, communication pathway, etc.

Note that with the example provided above, as well as numerous other examples provided herein, interaction may be described in terms of two, three, or four network elements. However, this has been done for purposes of clarity and example only. In certain cases, it may be easier to describe one or more of the functionalities of a given set of flows by only referencing a limited number of network elements. It should be appreciated that topologies illustrated in and described with reference to the accompanying FIGURES (and their teachings) are readily scalable and may accommodate a large number of components, as well as more complicated/sophisticated arrangements and configurations. Accordingly, the examples provided should not limit the scope or inhibit the broad teachings of the illustrated topologies as potentially applied to myriad other architectures.

It is also important to note that the steps in the preceding flow diagrams illustrate only some of the possible signaling scenarios and patterns that may be executed by, or within, communication systems shown in the FIGURES. Some of these steps may be deleted or removed where appropriate, or these steps may be modified or changed considerably without departing from the scope of the present disclosure. In addition, a number of these operations have been described as being executed concurrently with, or in parallel to, one or more additional operations. However, the timing of these operations may be altered considerably. The preceding operational flows have been offered for purposes of example and discussion. Substantial flexibility is provided by communication systems shown in the FIGURES in that any suitable arrangements, chronologies, configurations, and timing mechanisms may be provided without departing from the teachings of the present disclosure.

Although the present disclosure has been described in detail with reference to particular arrangements and configurations, these example configurations and arrangements may be changed significantly without departing from the scope of the present disclosure. For example, although the present disclosure has been described with reference to particular communication exchanges, embodiments described herein may be applicable to other architectures.

Numerous other changes, substitutions, variations, alterations, and modifications may be ascertained to one skilled in the art and it is intended that the present disclosure encompass all such changes, substitutions, variations, alterations, and modifications as falling within the scope of the appended claims. In order to assist the United States Patent and Trademark Office (USPTO) and, additionally, any readers of any patent issued on this application in interpreting the claims appended hereto, Applicant wishes to note that the Applicant: (a) does not intend any of the appended claims to invoke paragraph six (6) of 35 U.S.C. section 142 as it exists on the date of the filing hereof unless the words "means for" or "step for" are specifically used in the particular claims; and (b) does not intend, by any statement in the specification, to limit this disclosure in any way that is not otherwise reflected in the appended claims.

## Claims

1. A method for reconstructing electrocardiogram (ECG) system signals for a human subject, the method comprising:
recording first signals acquired by a 12-lead standard ECG system;
recording second signals acquired by an *M*-lead system; and
training, using a portion of the recorded first signals and a portion of the recorded second signals, a machine learning model to produce reconstructed 12-lead standard ECG system signals using the *M*-lead system, wherein the machine learning model comprises an artificial neural network (ANN) including multiple inputs corresponding to leads of the *M*-lead system.

2. The method of claim 1, wherein the leads of the *M*-lead system comprise a subset of leads of an enhanced ECG system, and wherein the enhanced ECG system includes the 12-lead standard ECG system and at least one additional electrode.

3. The method of claim 1 or claim 2, further comprising producing reconstructed 12-lead standard ECG system signals by applying the machine learning model to a second portion of the recorded second signals.

4. The method of any of claims 1 to 3, further comprising evaluating an accuracy of the machine learning model by comparing a second portion of the recorded first signals with the reconstructed 12-lead standard ECG system signals.

5. The method of any of claims 1 to 3, further comprising,
evaluating an accuracy of the machine learning model by determining whether intrinsic characteristics of the first signals are met by the reconstructed 12-lead standard ECG system signals; and
assigning a confidence value to the machine learning model based on results of the evaluating.

6. The method of claim 1, wherein a portion of the recorded first signals and a portion of the recorded second signals are used to train coefficients of the ANN.

7. The method of claim 1, wherein the ANN comprises a multilayer perceptron (MLP) network comprising one or more hidden layers, each having one or more neurons; and/or
wherein the ANN comprises a multiple output ANN; and/or
wherein the ANN comprises multiple single output ANNs.

8. The method of any of claims 1 to 7, wherein the ANN comprises at least one additional input corresponding to at least one of an angle of a cardiac vector, a magnitude of the cardiac vector, and information regarding the human subject.

9. The method of any one of claim 7, wherein the multiple single output ANNs are separated into multiple groups of 12 ANNs, with the multiple groups of 12 ANNs representing a committee of experts.

10. An electrocardiogram (ECG) reconstruction system comprising:
a plurality of electrodes comprising a 12-lead standard ECG system, wherein the plurality of electrodes is configured to be applied to a human subject;
a training module configured to use first signals acquired by the 12-lead standard ECG system and second signals acquired by an *M*-lead system to train a machine learning model for reconstruction of 12-lead standard ECG system signals from the second signals, wherein the machine learning model comprises an artificial neural network (ANN) including multiple inputs corresponding to leads of the M-lead system; and
a reconstruction module configured to use the machine learning model to reconstruct the 12-lead standard ECG system signals using the *M*-lead system.

11. The ECG reconstruction system of claim 10, wherein the leads of the *M*-lead system comprise a subset of leads of an enhanced ECG system, and wherein the enhanced ECG system includes the 12-lead standard ECG system and at least one additional electrode.

12. The ECG reconstruction system of claim 10 or claim 11, further comprising an evaluation module configured to evaluate an accuracy of the machine learning model by comparing the first signals with the reconstructed signals.

13. The ECG reconstruction system of any of claims 10 to 12, wherein the ANN comprises a multiple output ANN; and/or
wherein the ANN comprises multiple single output ANNs.

14. The ECG reconstruction system of claim 13, wherein the multiple single output ANNs are separated into multiple groups of 12 ANNs, with the multiple groups of 12 ANNs representing a committee of experts.

15. The ECG reconstruction system of any of claims 10 to 14, wherein a number of leads of the M-lead system is equal to three leads.
